(19) Europäisches Patentamt
European Patent Office
Office européen des brevets

(11) EP 3 257 849 A1

(12) EUROPEAN PATENT APPLICATION

(43) Date of publication:
20.12.2017 Bulletin 2017/51

(21) Application number: 16174440.4

(22) Date of filing: 14.06.2016

(51) Int Cl.:
C07D 403/04 (2006.01)
C07D 405/04 (2006.01)
C07D 409/04 (2006.01)
C07D 495/04 (2006.01)
C07D 495/14 (2006.01)
C07D 209/86 (2006.01)
C07D 209/88 (2006.01)
H01L 51/05 (2006.01)
H01L 51/30 (2006.01)
H01L 51/40 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: Solvay SA
1120 Bruxelles (BE)

(72) Inventors:
- FENOLL, Mathieu
3078 Everberg (BE)
- VAN BEVEREN, Maxime
6032 Mont-Sur-Marchienne (BE)

# (54) ORGANIC SEMICONDUCTOR COMPOSITION AND SEMICONDUCTING LAYER OBTAINED THEREFROM

(57) The present invention concerns a composition comprising at least one crystalline organic semiconductor and at least one compound of formula

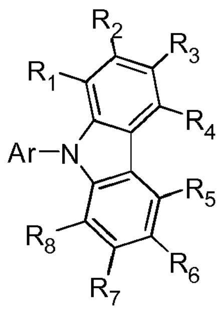

wherein $R_1$, $R_2$, $R_3$, $R_4$, $R_5$, $R_6$, $R_7$ and $R_8$, which may be the same or different, represent H or a $C_1$-$C_{50}$ hydrocarbyl or heterohydrocarbyl group, and wherein Ar is an optionally substituted aryl or heteroaryl group.

EP 3 257 849 A1

## Description

The present invention concerns an organic semiconductor composition comprising a crystalline semiconductor and at least one compound bearing at least one optionally substituted carbazole moiety and the use thereof for the manufacture of organic semiconducting layers suitable for the manufacture of organic electronic devices.

Organic semiconducting layers have been the object of many attentions in the field of organic electronics. Especially when low cost organic electronics devices are targeted, one needs cheap material, easy processes and high cadences to produce the devices. Process to manufacture organic semiconducting thin layers may involve deposition under vacuum or may involve a solution process like ink printing. Liquid coating of organic semiconductor composition is generally preferred over vacuum deposition techniques. The use of crystalline semiconductor such as TIPS-pentacene alone in a semiconducting layer may require drastic conditions and long times to promote convenient crystallization both being detrimental to manufacture with high cadences. Moreover, crystallization of any crystalline OSC alone is such a sensitive process that reproducibility may be impaired by minor changes during manufacturing.

Some prior art documents disclose the presence of likely amorphous carbazole containing compounds in OFET semiconducting layers.

WO16014980 relates to an organic electroluminescent transistor wherein the emissive layer is composed of a small molecule host which may be a compound bearing carbazole moieties and an Iridium complex. Nothing is taught about charge mobility within this layer.

US2010200841 proposes a method to prepare an organic layer by using a liquid composition comprising a small molecule organic semiconductor bearing carbazole moieties. This layer may be involved in an OFET in the semiconducting layer.

Compounds that contain carbazole moiety whether fused with another moiety, as it is the case for 5,11-dihydroindolo[3,2-b]carbazole or 5,12-dihydrocarbazolo[3,2-b]carbazole, whether grafted to a core, as it is the case for 2,5-di(9H-carbazol-3-yl)thiophene, may be crystalline semiconductors well suited for the manufacture of organic semiconducting layers in OFET.

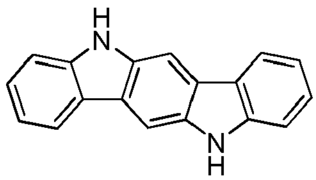
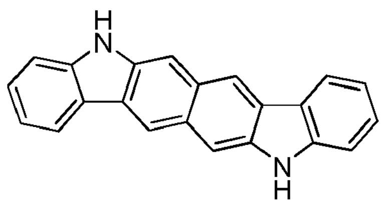

5,11-dihydroindolo[3,2-*b*]carbazole 5,12-dihydrocarbazolo[3,2-*b*]carbazole

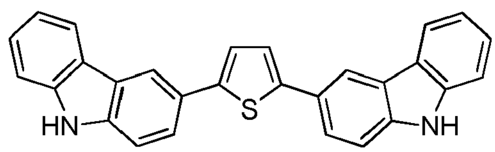

2,5-di(9*H*-carbazol-3-yl)thiophene

Jiang et al, Advanced materials (2011), 23(43), p.5075-5080, proposes an OFET device comprising a semiconducting layer made by physical vapour deposition of indolo[3,2-b]carbazole. Performances have been measured onto single crystal.

Crystalline layers of indolo[3,2-b]carbazole derivatives are grown by thermal sputtering by Svetlichnyi et al., Semiconductors, (2010), 44(12, p.1581-1587, to manufacture OFET having moderate performances.

Boudreault et al., J.Mater.Chem. (2009), 19, p.2921-2928, deposites 3,9-disubstituted indolo[3,2-b]carbazole under vacuum to prepare OFET having low to moderate properties.

Li et al., Advanced Materials (2005), 17(7), p.849-853, manufactures OFET comprising 5,11-dialkyl indolo[3,2-b]carbazole semiconducting layer obtained by vacuum evaporation. While device performances are relatively low, OFET prepared by solution process gives even less promising results.

Boudreault et al., Organic Electronics (2010), 11(10), p.1649-1659, prepare film of soluble indolo[3,2-b]carbazole derivatives by drop casting or solution shearing technique. Depending on the length of the molecules and of the number of alkyl chains that substitute the molecule, the film is amorphous or crystalline. Crystalline films give obviously the best performances in term of charge mobility. However, nothing is told about reproducibility of the processes.

Park et al., Advanced Materials (2013), 25(24), p.3351-3356 relates to OFET devices, which comprise indolocarbazole derivatives made by solution process. The carbazole derivatives, which are crystalline OSC, are used herein as the sole component in the semiconducting layer. The preparation of said layer, which requires single crystals, is a slow evaporation process non compatible with high cadences of production.

Li et al, Organic Electronics, (2009), 10(5), p.910-917, proposes thiophene based materials bearing carbazole moieties to prepare, by vacuum deposition or by solution process, semiconducting layers having weak performances in OFET.

Another aspect of the use of carbazole containing compounds in semiconducting layers is the role of binder played when it is mixed with a crystalline OSC.

WO2005/055248 discloses OFET devices comprising a semiconducting layer comprising a crystalline pentacene derivative and a poly(9-vinylcarbazole) binder. The polymer is expected to promote easy processing, film uniformity and further device reliability. However, despite the fact that relatively high mobility of OFET is measured, the standard deviation is large as a % of a mean value and consequently reproducibility of the devices is questionable.

There is a need for a new organic semiconductor composition suitable for preparing a semiconducting layer of an electronic device having reproducible properties, in particular high field effect mobility.

There is also a need for an organic semiconducting layer having high stability and, more particularly, bias stress stability.

There is also a need for an organic semiconductor composition suitable for preparing semiconducting layer having high field effect mobility, low cost and easy processing.

The needs previously described and others, are advantageously met by a composition comprising at least one crystalline organic semiconductor and at least one compound bearing at least one optionally substituted carbazole moiety, said compound corresponding to formula (a)

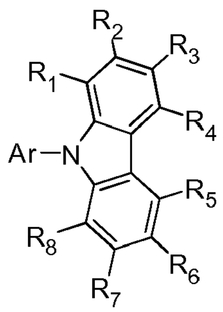

**Formula (a)**

and said compound being free of any spirobifluorene moiety corresponding to formula (b)

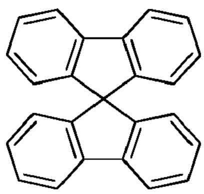

**Formula (b)**

wherein:

$R_1$, $R_2$, $R_3$, $R_4$, $R_5$, $R_6$, $R_7$ and $R_8$, which may be the same or different, represent H or a $C_1$-$C_{50}$ hydrocarbyl or heterohydrocarbyl group, and

Ar is an optionally substituted aryl or heteroaryl group.

The term "hydrocarbyl" as used herein refers to a group only containing carbon and hydrogen atoms. The hydrocarbyl group may be saturated or unsaturated, linear, branched or cyclic. If the hydrocarbyl is cyclic, the cyclic group may be an aromatic or non-aromatic group.

The term "heterohydrocarbyl" as used herein refers to a hydrocarbyl group wherein one or more of the carbon atoms is/are replaced by a heteroatom, such as Si, S, N or O. Included within this definition are heteraromatic rings, i.e. wherein one or more carbon atom within the ring structure of an aromatic ring is replaced by a heteroatom.

**Nature of Ar**

Ar is generally a substituted or unsubstituted aryl or a substituted or unsubstituted heteroaryl group, said aryl or heteroaryl group being derived from a compound selected from the list of compounds of following formulae:

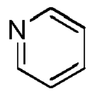
pyridine

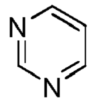
pyrimidine

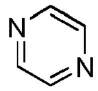
pyrazine

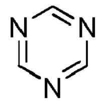
1,3,5-triazine

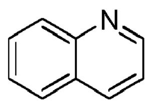
quinoline

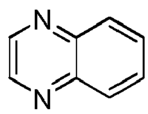
quinoxaline

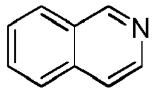
isoquinoline

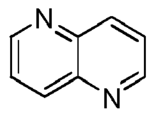
1,5-naphthyridine

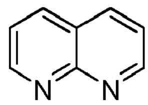
1,8-naphthyridine

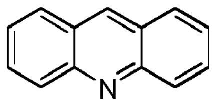
acridine

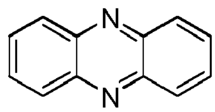
phenazine

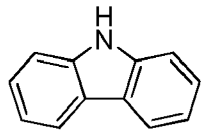
9*H*-carbazole

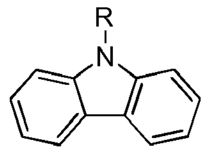
9-R-9*H*-carbazole

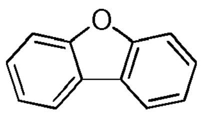
dibenzo[*b*,*d*]furan

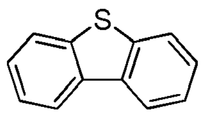
dibenzo[*b*,*d*]thiophene

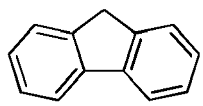
9*H*-fluorene

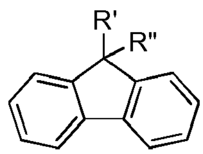
9-R'-9-R"-9H-fluorene

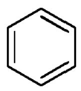
benzene

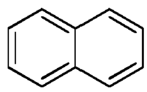
naphthalene

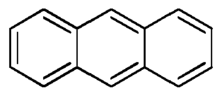
anthracene

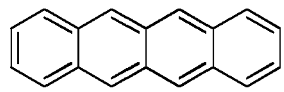
tetracene

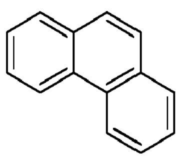
phenanthrene

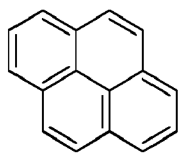
pyrene

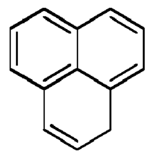
1*H*-phenalene

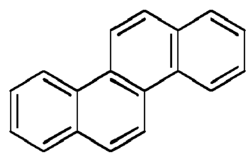
chrysene

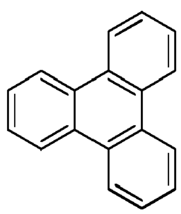
triphenylene

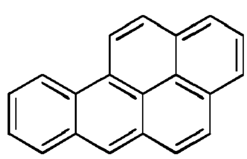
benzo[*pqr*]tetraphene

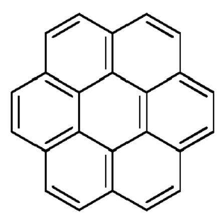
coronene

which compounds may be substituted or unsubstituted and, wherein R, R' and R" represent a $C_1$-$C_{24}$ hydrocarbyl or

heterohydrocarbyl group.

By aryl or heteroaryl group derived from a compound comprising at least one aromatic or heteroaromatic ring, is meant aryl or heteroaryl group obtainable by the removal of one hydrogen atom from the aromatic or heteroaromatic ring of the compound. For example, dibenzo[b,d]thiophen-2-yl can be obtained by removing the hydrogen atom in the position 2 of dibenzo[b,d]thiophene. Thus in the meaning of the present invention, dibenzothiophen-2-yl is an aryl group derived from dibenzothiophene.

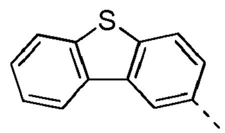

dibenzo[*b*,*d*]thiophen-2-yl

Ar may be substituted on any carbon atom with one or more substituent(s) X, wherein X may be the same or different at each occurrence. When Ar is substituted with at least one substituent X, X is advantageously chosen from halogen, $-NR_9R_{10}$ and $C_1$-$C_{10}$ hydrocarbyl or heterohydrocarbyl groups, wherein $R_9$ and $R_{10}$, which may be similar or different, are phenyl groups optionally substituted by at $C_1$-$C_{10}$ hydrocarbyl or heterohydrocarbyl group.

Examples for $C_1$-$C_{10}$ hydrocarbyl or $C_1$-$C_{10}$ heterohydrocarbyl groups are alkyl, silyl, alkyl silyl, alkoxy, cyano, cyanoalkyl, alkenyl, alkynyl, arylalkyl, aryl, heteroarylalkyl and heteroaryl groups. Preferred $C_1$-$C_{10}$ hydrocarbyl or heterohydrocarbyl group are alkyl, cyanoalkyl and heteroaryl group derived from 1,3,4-oxadiazole or carbazole compounds, wherein the 1,3,4-oxadiazole and carbazole compounds may be unsubstituted or substituted.

When X is a $C_1$-$C_{10}$ heterohydrocarbyl group derived from an optionally substituted carbazole, it is generally corresponding to the formula (c) below, $R_1$-$R_8$ being as previously defined

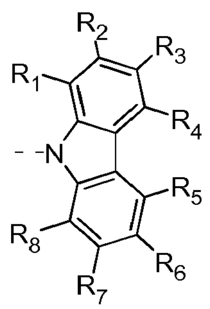

**Formula (c)**

In formula (c) specifically, all $R_i$ (i=8) may be chosen from hydrogen, $C_1$-$C_4$ alkyls and $C_1$-$C_4$ oxyalkyls, with the preference being given to hydrogen.

Advantageously, Ar is derived from a compound selected from the list consisting of dibenzo[b,d]furan, dibenzo[b,d]thiophene, carbazole, fluorene, benzene, naphthalene, anthracene and phenanthrene, which compounds may be substituted or unsubstituted.

Preferably, Ar is derived from dibenzo[b,d]furan , dibenzo[b,d]thiophene, fluorene or benzene, which compounds may be substituted or unsubstituted.

More preferably, Ar is derived from dibenzo[b,d]thiophene or benzene, which compounds may be substituted or unsubstituted.

Even more preferably, Ar is a phenyl group which may be unsubstituted or substituted.

**Nature of $R_1$ to $R_8$**

Preferably, at least one of $R_1$ to $R_8$ is a heterohydrocarbyl group derived from a compound chosen from dibenzo[b,d]furan, dibenzo[b,d]thiophene and carbazole; the compound from which the heterohydrocarbyl group is derived is preferably carbazole.

More preferably, at least two of $R_1$ to $R_8$ is a heterohydrocarbyl group derived from a compound chosen from dibenzo[b,d]furan, dibenzo[b,d]thiophene and carbazole; the compound from which the heterohydrocarbyls groups are derived is preferably carbazole.

Still more preferably, two and only two of $R_1$ to $R_8$ is a heterohydrocarbyl group derived from a compound chosen from dibenzo[b,d]furan, dibenzo[b,d]thiophene and carbazole; the compound from which both heterohydrocarbyls

groups are derived is preferably carbazole.

Among all $R_i$ (i=1 to 8), $R_3$ and/or $R_6$ is/are usually the $R_i$ which is/are preferably heterohydrocarbyl group(s) derived from a compound chosen from dibenzo[b,d]furan, dibenzo[b,d]thiophene and carbazole; ; the compound from which the one or more heterohydrocarbyl(s) group(s) is/are derived is preferably carbazole.

Besides, preferably at least two, more preferably at least 4, still more preferably at least 6 of $R_1$ to $R_8$ are chosen from hydrogen, $C_1$-$C_4$ alkyls and $C_1$-$C_4$ oxyalkyls, with the preference being given to hydrogen.

Among all $R_i$ (i=1 to 8), $R_1$, $R_2$, $R_4$, $R_5$, $R_7$ and/or $R_8$ are generally the $R_i$ which are preferably chosen from hydrogen, $C_1$-$C_4$ alkyls and $C_1$-$C_4$ oxyalkyls, with the preference being given to hydrogen.

## Embodiment E1

In a preferred embodiment E1, Ar is a phenyl group which is either unsubstituted or which is substituted by at least one substituent X as previously defined.

In embodiment E1, Ar is a phenyl group which is either unsubstituted or which is substituted by one and only one substituent X as previously defined.

When present, the one or more substituent(s) X of Ar is/are preferably chosen from X is chosen from halogen and $C_1$-$C_8$ heterohydrocarbyl group, more preferably from $C_1$-$C_6$ cyanoalkyls and $C_1$-$C_6$ heterohydrocarbyl electron-withdrawing groups such as oxadiazolyls and alkyloxadiazolyls wherein the alkyl substituent of the alkyloxadiazolyls comprises from 1 to 4 carbon atoms.

In embodiment E1, most preferred $R_1$, $R_2$, $R_4$, $R_5$, $R_7$ and $R_8$ are hydrogen and most preferred $R_3$ and $R_6$ are heterohydrocarbyl group(s) derived from a compound chosen from dibenzo[b,d]furan, dibenzo[b,d]thiophene and carbazole; the compound from which the one or more heterohydrocarbyl(s) group(s) is/are derived is preferably carbazole.

In embodiment E1, even most preferred $R_1$, $R_2$, $R_4$, $R_5$, $R_7$ and $R_8$ are hydrogen and even most preferred $R_3$ and $R_6$ are unsubstituted carbazol-9-yl groups.

## Embodiment E2

In a preferred embodiment E2, Ar is a dibenzo[b,d]furanyl group which is substituted by at least one X substituent as previously defined.

Ar is preferably dibenzo[b,d]furan-2-yl which is substituted by at least one X substituent as previously defined.

Ar is more preferably, dibenzo[b,d]furan-2-yl which is substituted in position 8 by a substituent X; the case being, a representation of the compound bearing at least one optionally substituted carbazole moiety according to embodiment E2 is provided by formula (d)

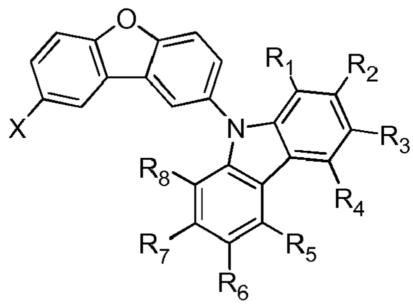

**Formula (d)**

wherein in formula (d) $R_1$, $R_2$, $R_3$, $R_4$, $R_5$, $R_6$, $R_7$, $R_8$ and X are as defined above.

In formula (d), X is preferably a $C_1$-$C_{30}$ heterohydrocarbyl group corresponding to the formula (c) below,

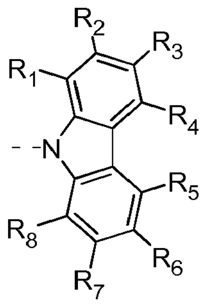

**Formula (c)**

wherein in formula (c) $R_1$, $R_2$, $R_3$, $R_4$, $R_5$, $R_6$, $R_7$ and $R_8$ are as defined above.

In formulae (d) and (c), $R_1$, $R_2$, $R_3$, $R_4$, $R_5$, $R_6$, $R_7$ and $R_8$ are preferably chosen from hydrogen, $C_1$-$C_4$ alkyls and $C_1$-$C_4$ oxyalkyls, and are more preferably hydrogen.

**Embodiment E3**

In a preferred embodiment E3, Ar is a dibenzo[b,d]thiophenyl group which is substituted by at least one X substituent as previously defined.

Ar is preferably dibenzo[b,d]thiophen-2-yl which is substituted by at least one X substituent as previously defined.

Ar is more preferably dibenzo[b,d]thiophen-2-yl which is substituted in position 8 by a substituent X; the case being a representation of the compound bearing at least one optionally substituted carbazole moiety according to the preferred embodiment E3 is provided by formula (e)

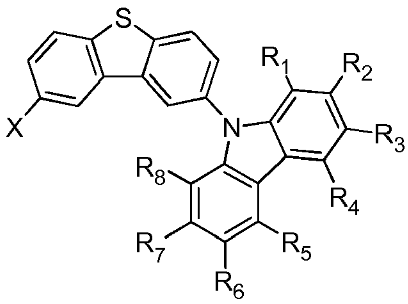

**Formula (e)**

wherein in formula (e) $R_1$, $R_2$, $R_3$, $R_4$, $R_5$, $R_6$, $R_7$, $R_8$ and X are as defined above.

In formula (e), X is preferably a $C_1$-$C_{30}$ heterohydrocarbyl group corresponding to the formula (c) below

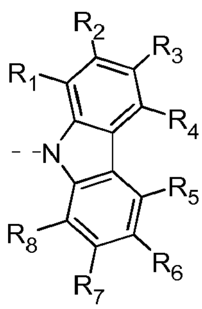

**Formula (c)**

wherein in formula (c) $R_1$, $R_2$, $R_3$, $R_4$, $R_5$, $R_6$, $R_7$ and $R_8$ are as defined above.

In formulae (e) and (c), $R_1$, $R_2$, $R_3$, $R_4$, $R_5$, $R_6$, $R_7$ and $R_8$ are preferably chosen from hydrogen, $C_1$-$C_4$ alkyls and $C_1$-$C_4$ oxyalkyls, and are more preferably hydrogen.

**Embodiment E4**

In a preferred embodiment E4, Ar is a fluorenyl group which is substituted by at least one X substituent as previously defined.
Ar is preferably fluorene-3-yl which is substituted by at least one X substituent as previously defined.
Ar is more preferably, fluorene-3-yl which is substituted in position 6 by a substituent X; the case being a representation of the compound bearing at least one optionally substituted carbazole moiety according to embodiment E4 is provided by formula (f)

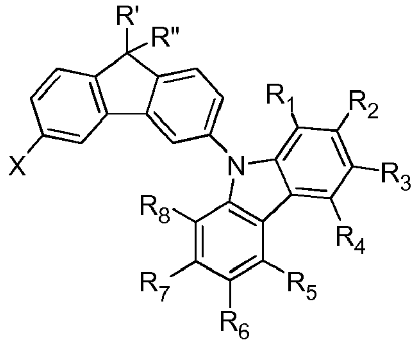

**Formula (g)**

wherein R', R", $R_1$, $R_2$, $R_3$, $R_4$, $R_5$, $R_6$, $R_7$, $R_8$ and X are as defined above.
In formula (f), R' and R", independently from each other, are preferably chosen from phenyl, $C_1$-$C_{12}$ alkyls and $C_1$-$C_{12}$ oxyalkyls, and are more preferably $C_1$-$C_4$ alkyls.
In formula (f), X is preferably a $C_1$-$C_{30}$ heterohydrocarbyl group corresponding to the formula (c) below,

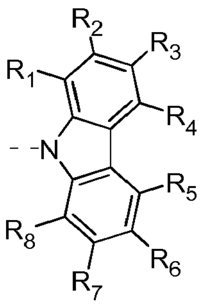

**Formula (c)**

wherein in formula (c) $R_1$, $R_2$, $R_3$, $R_4$, $R_5$, $R_6$, $R_7$ and $R_8$ are as defined above.
In formulae (f) and (c), $R_1$, $R_2$, $R_3$, $R_4$, $R_5$, $R_6$, $R_7$ and $R_8$ are preferably chosen from hydrogen, $C_1$-$C_4$ alkyls and $C_1$-$C_4$ oxyalkyls, and are more preferably hydrogen.

**Binder**

In the composition according to the invention, the at least one compound bearing at least one optionally substituted carbazole moiety is advantageously used as binder.
Non exhaustive examples of compounds bearing at least one optionally substituted carbazole moiety particularly well suited to be used in the composition of the present invention are represented in the formulae (I) to (XXXVI) below

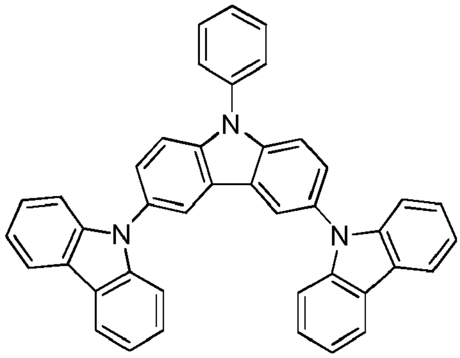

(I)

**Binder 1**

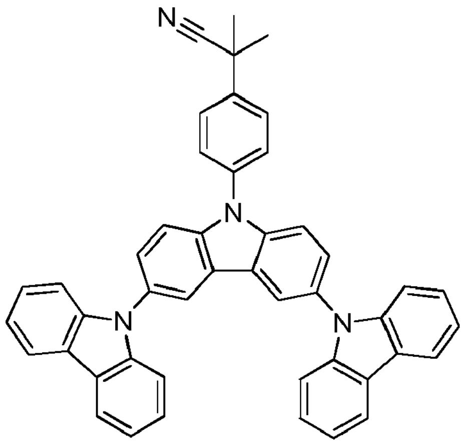

(II)

**Binder 2**

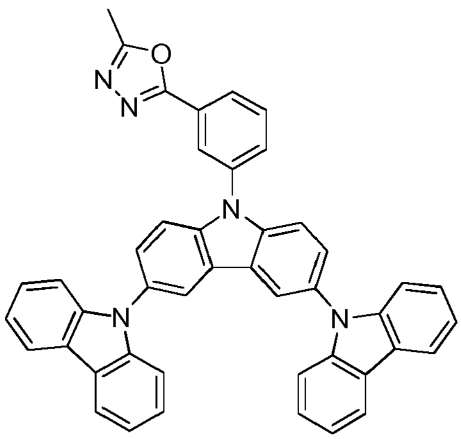

(III)

**Binder 3**

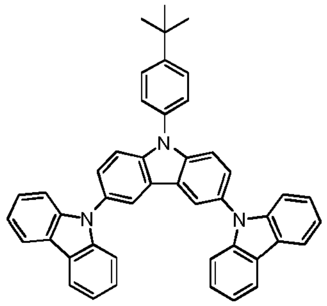
(IV)

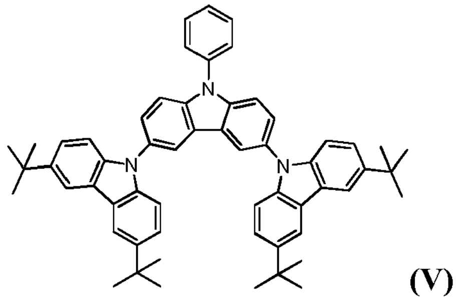
(V)

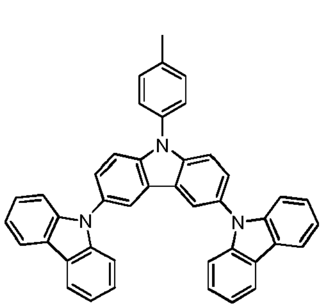
(VI)

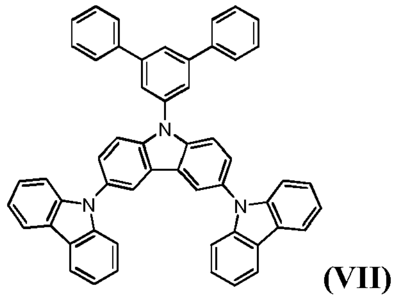
(VII)

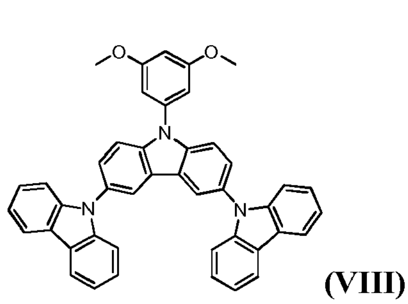
(VIII)

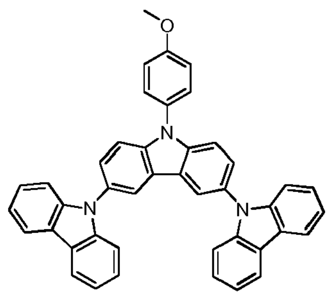
(IX)

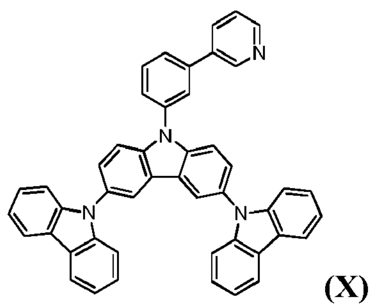
(X)

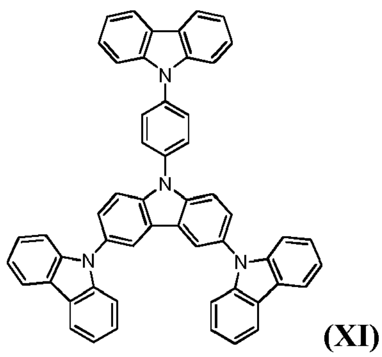
(XI)

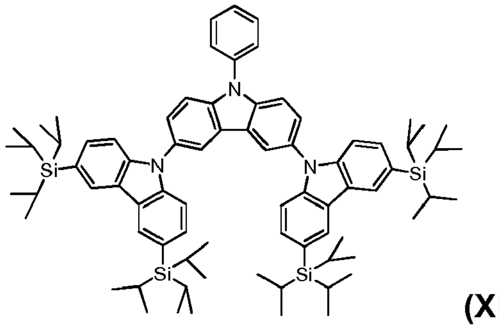
(XII)

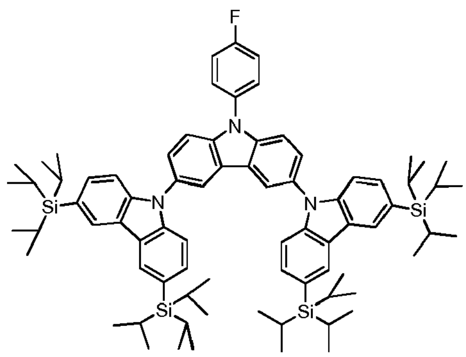

(XIII)

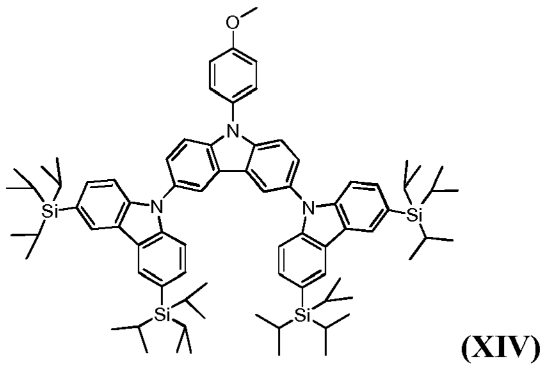

(XIV)

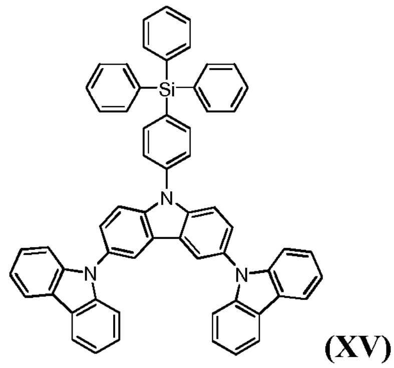

(XV)

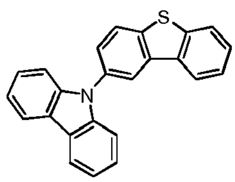

(XVI)

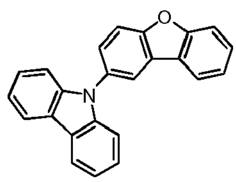

(XVII)

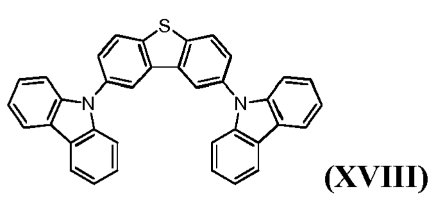

(XVIII)

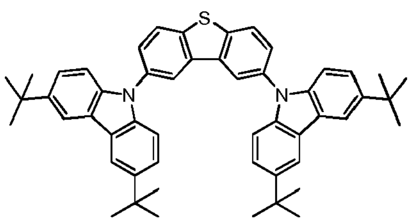

(XIX)

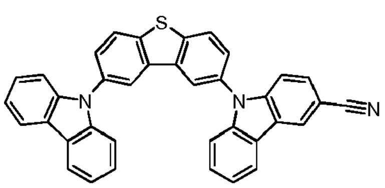

(XX)

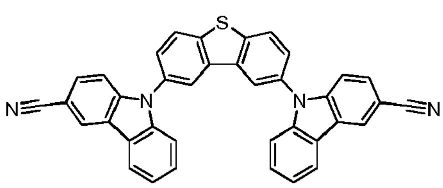

(XXI)

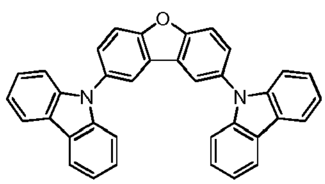

(XXII)

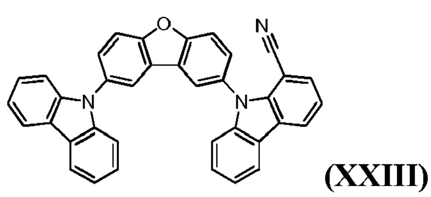

(XXIII)

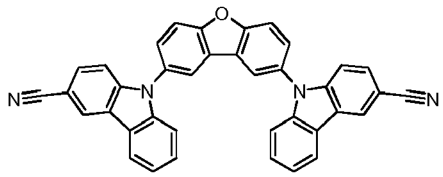

**(XXIV)**

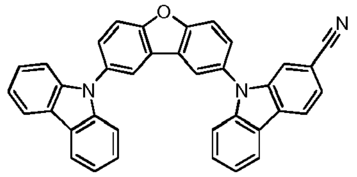

**(XXV)**

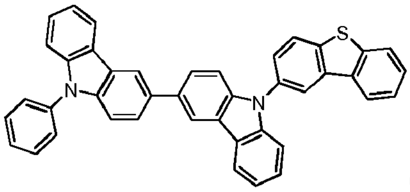

**(XXVI)**

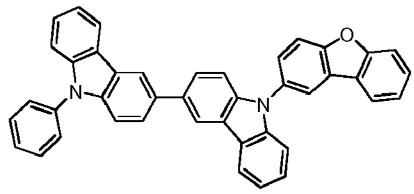

**(XXVII)**

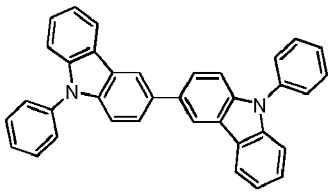

**(XXVIII)**

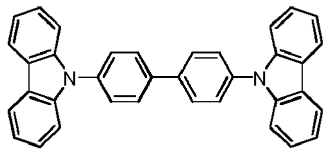

**(XXIX)**

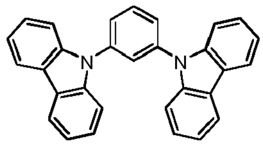

**(XXX)**

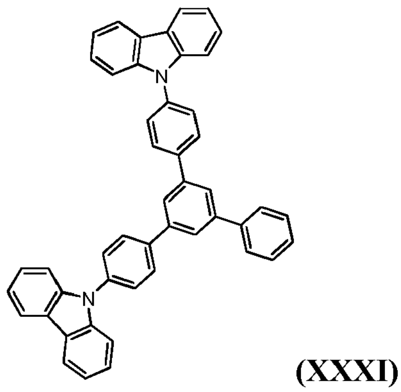

**(XXXI)**

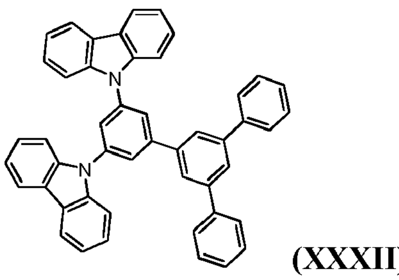

**(XXXII)**

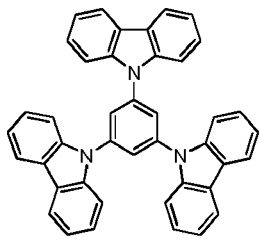

**(XXXIII)**

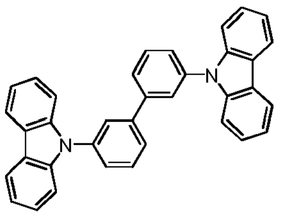

(XXXIV)

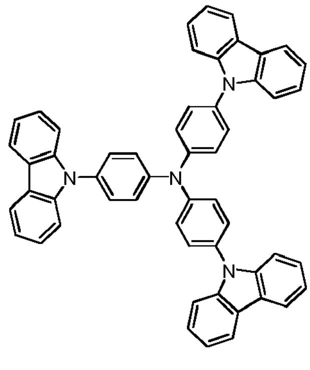

(XXXV)

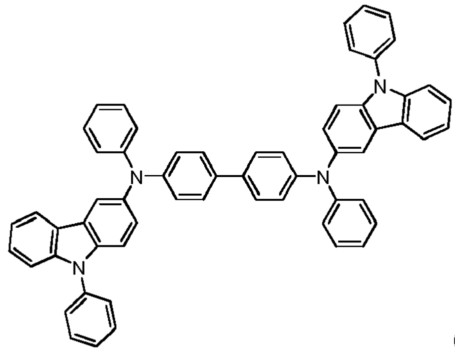

(XXXVI)

The compound bearing at least one optionally substituted carbazole moiety according to the invention is advantageously an amorphous material. It exhibits advantageously a glass transition temperature of at least 80°C, preferably of at least 100°C and even more preferably of at least 120°C.

It has been advantageous, in certain circumstances, that the compound bearing carbazole moiety used in the compositions of the present invention has a HOMO level of less than -5.0 eV, preferably less than -5.2 eV and more preferably a HOMO level of less than -5.5 eV; besides, it has a LUMO level of more than -3.5 eV, preferably more than -3.3 eV and more preferably of more than -3.0 eV.

The organic semiconductor composition in accordance with the invention comprises at least one crystalline organic semiconductor. By crystalline, is meant a compound that spontaneously self-organizes to give material which reveals a melting point that can be measured by any technique known by a person having ordinary skill in the art. For example, the presence of a melting point and its value can be determined by Differential Scanning Calorimetry. The crystalline organic semiconductor is chosen such that it exhibits a high tendency to crystallize when solution coating methods are used.

The crystalline semiconductor is preferably a small molecule which has a molecular weight of 1600 Daltons or less, preferably 1200 Daltons or less, more preferably 1000 Daltons or less, even more preferably 800 Daltons or less.

Preferred crystalline organic semiconducting materials which can be used in the present invention have a field effect mobility of at least 0.01 $cm^2/Vs$, preferably of at least 0.1 $cm^2/Vs$, more preferably of at least 1 $cm^2/Vs$. The mobility referred to here is preferably determined in the saturation mode using a field effect transistor. In this technique, for each fixed gate voltage $V_{GS}$ the drain-source voltage $V_{DS}$ is increased until the current $I_o$ saturates. Next, the square root of this saturated current is plotted against the gate voltage and the slope $m_{sat}$ is measured. Then the mobility is

$$\mu = m_{sat}^2\ 2L/WC_i \qquad \text{(Equation 1)}$$

where L and W are the length and width of the channel and $C_i$ is the gate insulator capacitance per unit area.

There is no limitation in the choice of the structure of the crystalline organic semiconductor that can be used in the present invention. The person skilled in the art can select the crystalline organic semiconductor based on his professional knowledge and based on some reviews on the subject. Crystalline organic semiconductors having suitable structures can be found in the review of Daoben Zhu et al. in Chemical Reviews, 2012, 112, 2208-2267 and more particularly in Table 2 of the said review.

Exemplary small molecule organic semiconductors are selected from compounds of formulae:

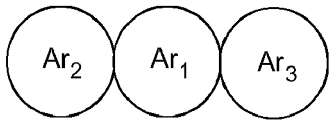

(1)

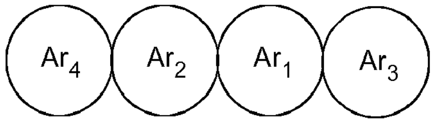

(2)

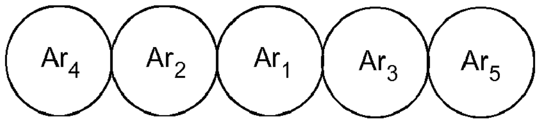

(3)

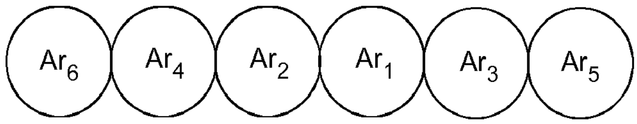

(4)

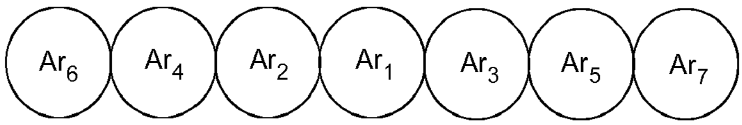

(5)

wherein $Ar_1$ to $Ar_7$ are the same or different at each occurrence and are each independently selected from the group consisting of monocyclic aromatic rings and monocyclic heteroaryl aromatic rings, with at least one of $Ar_1$ to $Ar_7$ being substituted with at least one substituent Z which may be the same or different at each occurrence and is selected from the group consisting of unsubstituted or substituted straight, branched or cyclic alkyl groups having from 1 to 50 carbon atoms, alkoxy groups having from 1 to 50 carbon atoms, aryloxy groups having from 6 to 40 carbon atoms, alkylaryloxy groups having from 7 to 40 carbon atoms, alkoxycarbonyl groups having from 2 to 40 carbon atoms, aryloxycarbonyl groups having from 7 to 40 carbon atoms, amino groups that may be unsubstituted or substituted with one or two alkyl groups having from 1 to 20 carbon atoms, each of which may be the same or different, amido groups, silyl groups that may be unsubstituted or substituted with one, two or three alkyl groups having from 1 to 20 carbon atoms, silylethinyl groups that may be unsubstituted or substituted with one, two or three alkyl groups having from 1 to 20 carbon atoms, alkenyl groups having from 2 to 20 carbon atoms, the carbamoyl group, the haloformyl group, the formyl group, the cyano group, the isocyano group, the isocyanate group, the thiocyanate group, the thioisocyanate group, OH, nitro, cyano, haloalkyl groups having 1 to 20 carbon atoms and wherein $Ar_1$, $Ar_2$ and $Ar_3$ may each optionally be fused to one or more further monocyclic aromatic or heteroaromatic rings.

In accordance with a preferred embodiment at least one of $Ar_1$ to $Ar_7$ comprises a 5 to 7 membered heteroaryl group containing from 1 to 3 sulfur atoms, oxygen atoms, selenium atoms and/or nitrogen atoms.

In a still further embodiment $Ar_1$ to $Ar_7$ are independently selected from phenyl and thiophene and at least one of $Ar_1$ to $Ar_7$ is thiophene.

A first group of small molecule organic semiconductors in accordance with the above general formulae which can be used in the compositions of the present invention are oligo-and polyacenes.

Acenes or polyacenes are a class of organic compounds and parties cyclic aromatic hydrocarbons made up of linearly fused benzene rings, i.e. having a skeleton of general formula

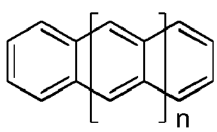

which may be substituted with substituents Z as defined above in any position. In preferred compounds of this group n is an integer in the range of from 1 to 4.

Pentacene compounds for which n is 3 and corresponding to formula (3) have been used in a number of applications for the manufacture of organic electronic devices. Exemplary representatives of suitable pentacene compounds are represented by formulae below:

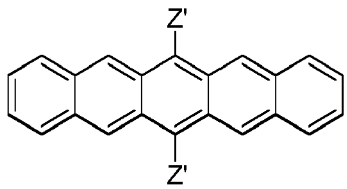

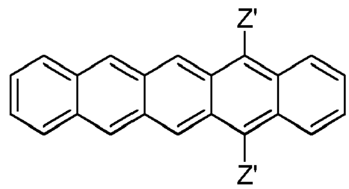

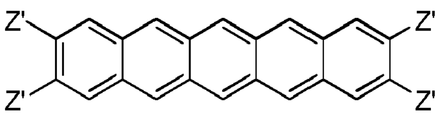

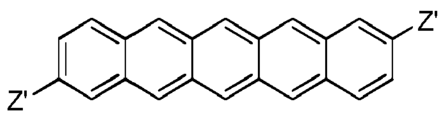

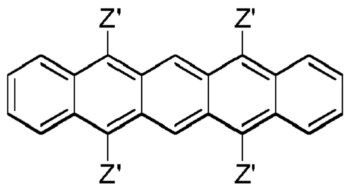

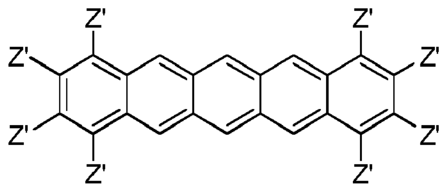

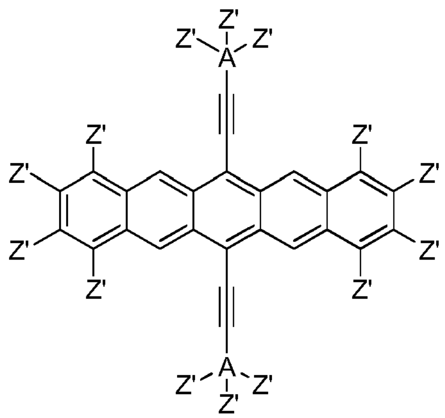

(6)

in which Z', which may be the same or different at each occurrence, is hydrogen or has a meaning as defined above for Z, with the proviso that at least one substituent Z' is different from hydrogen, and wherein A is carbon, silicon or germanium, preferably silicon or germanium. The substituents Z' at the atom A in this case are preferably alkyl groups or alkoxy groups having from 1 to 20, preferably from 1 to 12 and even more preferably 1 to 8 carbon atoms.

Still another group of compounds corresponding to formula (3) is represented by formula

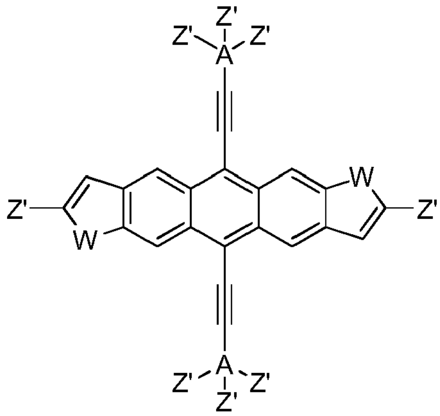

(7)

wherein again Z' and A are as defined above and may be the same or different at each occurrence and wherein W is selected from the group consisting of S, NH, NZ' or Se, preferably from S or Se. The substituents Z' at the atom A in this case are preferably alkyl groups or alkoxy groups having from 1 to 20, more preferably from 1 to 12 and even more preferably from 1 to 8 carbon atoms and the substituent Z' in NZ' is an alkyl group having from 1 to 12, more preferably from 1 to 8 carbon atoms.

Another group of compounds of general formula (2) preferred as organic semiconductors in the present invention are compounds of general formula

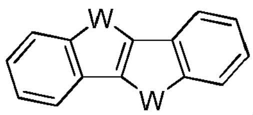

(8)

wherein W is as defined above and which may bear substituents Z' as defined above at any of the carbon atoms of the ring system.

Another group of compounds of general formula (4) preferred as organic semiconductors in the present invention are compounds of general formula

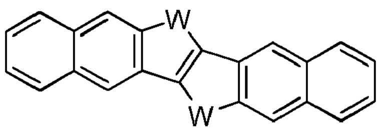

(9)

wherein W is as defined above and which may bear substituents Z' as defined above at any of the carbon atoms of the ring system.

Another group of compounds of general formula (5) preferred as organic semiconductors in the present invention are compounds of general formula

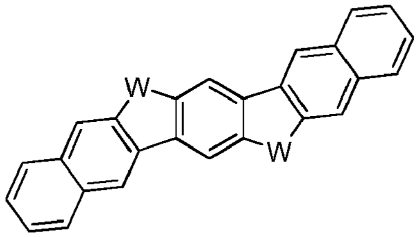

(10)

wherein W is as defined above and which may bear substituents Z' as defined above at any of the carbon atoms of the ring system.

Still another group of preferred organic semiconductors in the compositions of the present invention and corresponding to formula (3) are furan or thiophene V-shaped organic semiconducting materials of general formula

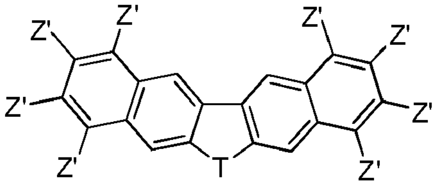

wherein T is an oxygen or a sulfur atom and Z', which may be the same or different at each occurrence, may have the meaning as defined above.

Representative examples are

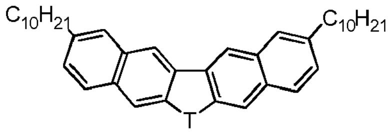

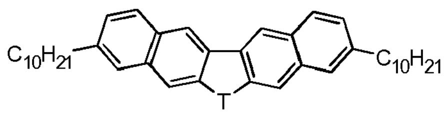

wherein T is preferably sulfur.

Compounds of this structure as well as their synthesis have been described in Adv.Mater. 2013, 25, 6392-6397 and Chem. Comm. 2014, 50, 5342-5344 to which reference is made for further details.

Still another group of preferred organic semiconductors in the compositions of the present invention and corresponding to formula (1) is V-shaped organic semiconducting materials of general formula

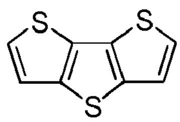

which may bear substituents Z' as defined above at any of the carbon atoms of the ring system.

Representative examples are

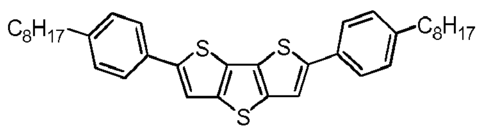

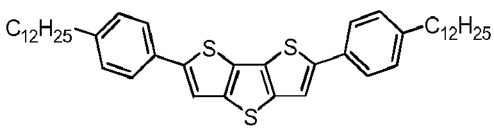

Compounds of this structure as well as their synthesis have been described in Chem. Comm. 2013, 49, 6483-6485 to which reference is made for further details.

Particularly preferred examples for compounds of formula (6) are the compounds of formulae below which are generally known under the names given next to the formula

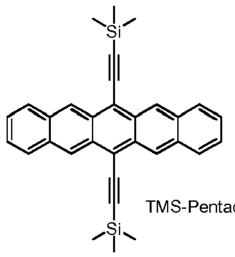

TMS-Pentacene

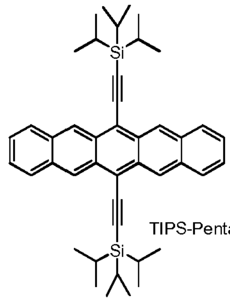

TIPS-Pentacene

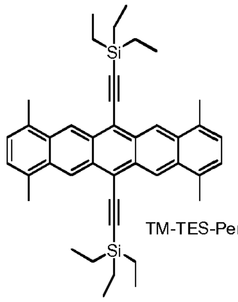

TM-TES-Pentacene

**(11)**

A preferred organic semiconductor corresponding to a general formula (7) is represented by formula

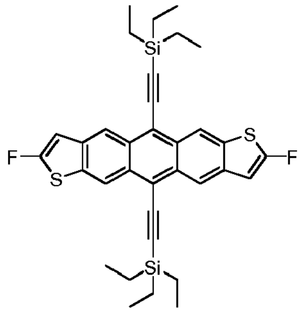

diF-TES-ADT

Particularly preferred representatives of general formula (8) are benzothieno benzothiophene derivatives represented by general formula below

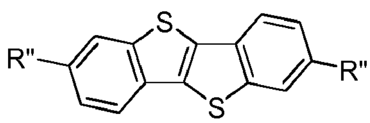

wherein R", which may be the same or different, represents an alkyl group having 1 to 30, preferably from 1 to 20 and most preferably from 1 to 12 carbon atoms. A particularly preferred compound of this group is known as C8-BTBT in which both substituents R" are n-C8H17 (n-octyl) groups.

Particularly preferred representative of general formula (9) is Dinaphtothienothiophene derivative represented by general formula below

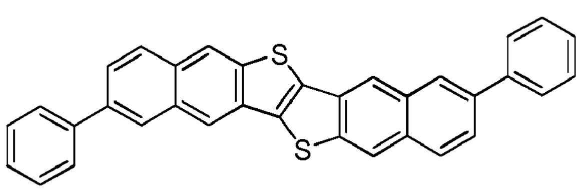

Particularly preferred representatives of general formula (10) are derivatives represented by general formula

below

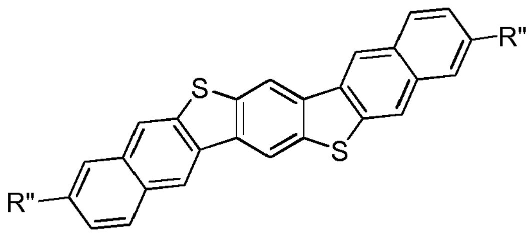

wherein R", which may be the same or different, represents an alkyl group having 1 to 30, preferably from 1 to 20 and most preferably from 1 to 12 carbon atoms. A particularly preferred compound of this group is known as C10-DNBDT in which both substituents R" are n-$C_{10}H_{21}$ groups.

In the composition according to the invention, the weight ratio of crystalline organic semiconductor to compound bearing carbazole moiety is comprised from 5/95 to 95/5, preferably from 25/75 to 75/25, more preferably from 34/66 to 66/34.

The composition according to the invention may be available in the form of an ink, i.e. it further comprises at least one organic solvent, which form makes it easily deposited onto a substrate.

In such case the combined content of the compound bearing carbazole moiety and of the crystalline organic semiconductor versus the total weight of the composition (i.e. including, inter alia, the organic solvent) is of at least 0.5 wt. %, preferably of at least 1 wt. % and more preferably of at least 1.5 wt. %; besides, the combined content of the compound bearing carbazole moiety and of the crystalline organic semiconductor versus the total weight of the composition (i.e. including, inter alia, the organic solvent) is of at most 10 wt. %, preferably of at most 5 wt. % and more preferably of at most 3 wt. %, with regard to the total weigh of the composition.

In particular embodiments, the composition is coated onto a substrate by vacuum deposition. Then, it is advantageously free of solvent. Besides, when the composition according to the invention is available in the form of an ink, the solvent comprised in the ink is advantageously removed from the composition after the ink is deposited on the substrate, thereby obtaining advantageously a composition essentially free or even completely free of solvent.

Thus, in the invented composition, an organic solvent may be present or not.

In any case, the weight content of the compound bearing carbazole moiety is generally of at least 5 wt. % with regard to the total weight of the composition diminished by the weight of the organic solvent, whatever present or not. It is preferably of at least 25 wt. %, more preferably of at least 35 wt. %, even more preferably of at least 50 wt. % with regard to the total weight of the composition diminished by the weight of the organic solvent. Besides, the weight content of the compound bearing carbazole moiety is generally of at most 95 wt. % with regard to the total weight of the composition diminished by the weight of the organic solvent; it is preferably of at most 85 wt. %, more preferably of at most 75 wt. %, even more preferably of at most 65 wt. % with regard to the total weight of the composition diminished by the weight of the organic solvent.

The viscosity of the solvents preferably is in the range of from 0.2 to 50 mPas, preferably in the range of from 1 to 20 mPas, measured at 25°C.

The Hansen solubility parameters of the solvent used, expressed in $MPa^{1/2}$, are preferably within the following ranges:

Hd: 15 to 25, preferably 17 to 23
Hp: 0 to 20, preferably 0 to 15
Hh: 0 to 25, preferably 0 to 20

wherein Hd, Hp and Hh respectively represent the dispersive, the polar and the hydrogen bonding solubility parameters.

The solvent surface tension in mN/m (at 20°C) is preferably in the range of from 10 to 70, more preferably in the range of from 20 to 50 and most preferably in the range of from 26 to 38.

Examples of suitable and preferred organic solvents include, without being limited to, dichloromethane, trichloromethane, monochlorobenzene, o-dichlorobenzene, the respective bromobenzenes, tetrahydrofuran, anisole, morpholine, toluene, o-xylene, m-xylene, p-xylene, the isomeric trimethylbenzenes, in particular 1,3,5 trimethylbenzene (also known as mesitylene), 1,4-dioxane, acetone, 1,1,2,2-tetrachloroethane, ethyl acetate, n-butyl acetate, dimethylformamide, dimethylacetamide, dimethylsulfoxide, tetralin (tetrahydronaphthalene), halogenated naphthalenes, such as 1-bromo naphthalene, decalin, indane and/or mixtures thereof.

Especially preferred solvents for use in the compositions according to the present invention are anisole, the isomeric xylenes, toluene, mesitylene, tetralin, chlorobenzene, bromobenzene, o-dichlorobenzene and 1-bromonaphthalin and/or mixtures thereof.

Even more preferred solvents are anisole and tetralin.

The preferred solvents have a boiling point in the range of from 80 to 350, preferably of from 100 to 300 and even more preferably of from 130 to 230°C.

The organic semiconductor composition in accordance with the present invention can additionally comprise one or more further components like e.g. surface-active compounds, lubricating agents, wetting agents, dispersing agents, hydrophobic agents, adhesive agents, flow improvers, defoaming agents, deaerators, diluents (reactive or non-reactive), auxiliaries, colorants, dyes, pigments or nanoparticles.

It is also the object of the present invention to disclose the use of the organic semiconductor composition in accordance with the present invention for the manufacture of a semiconducting layer.

The semiconducting layer obtained through the use of the organic semiconductor composition in accordance with the present invention is typically at most 1 μm thick, preferably at most 500 nm although greater thicknesses may be achieved if required. The semiconducting layer obtained is typically at least 10 nm thick, preferably at least 50 nm.

The present invention also relates to a process for the manufacture of an organic semiconducting layer comprising applying the composition as above described to form a thin layer onto a substrate

Preferably, in the invented process, the composition additionally comprises at least one organic solvent and the process further comprises evaporating the solvent from the layer.

Indeed, to form a layer onto a substrate, especially a thin layer, liquid coating of organic semiconductor composition is generally preferred over vacuum deposition techniques which are possible anyway. The organic semiconductor compositions in accordance with the present invention enable the use of a number of liquid coating techniques.

Just by way of example dip coating, spin coating, slot die coating, inkjet printing, letter-press printing, screen printing, doctor blade coating, roller printing, reverse roller-printing, offset lithography printing, flexographic printing, flexographic coating, spray coating, brush coating or pad printing may be mentioned here.

The inkjet fluid (i.e. the mixture of semiconducting compounds, binder and solvent) preferably has a viscosity at 20°C of 1-100 mPas, more preferably of 1-50 mPas and most preferably 1-30 mPas.

A substrate used for preparing the organic semiconducting layer may include any underlying device layer, electrode or separate substrate such as e.g. silicon wafer, glass or polymer substrate.

The present invention furthermore relates to an organic electronic device comprising an organic semiconducting layer in accordance with the invention. The electronic device generally comprises a gate electrode, a source electrode and a drain electrode, said device further comprises an organic semiconducting layer between the source and drain electrode, which organic semiconducting layer is obtained using an organic semiconductor composition in accordance with the present invention.

Preferred organic electronic devices are organic field effect transistors.

The present invention finally relates to an electronic device comprising an organic field effect transistor comprising a semiconducting layer obtained using an organic semiconductor composition in accordance with the invention.

The said electronic device is generally a display, more preferably an active matrix display, even more preferably an active matrix OLED display.

The following examples provide further information on the preferred embodiments of the present invention.

## Examples

Organic Thin Film Transistors (OTFTs) were fabricated in top gate and bottom contact configuration using glass based substrates as follows:

> 1" square glass substrates (ex: Corning XG) were cleaned using sonication bathes for 5 minutes in Deconex (3% in water) followed by rinsing in ultrapure water and dried using compressed air. A planarization layer was first spin coated on top of the cleaned substrates in order to create a 30 nm film after photocrosslinking. Au (30 nm) bottom contact source/drain (S/D) electrodes were deposited on top of the planarization layer by thermal evaporation through a shadow mask. The 16 transistors S/D consisted of electrodes with channel length of 50μm and channel width of 0.5mm. The substrates then underwent UV/$O_3$ treatment (model 42-220, from Jelight Company, Inc.), treatment time 5min. Prior to spin coating of the OSC solution, a 10mM solution of 4-fluorobenzenethiol in 2-propanol was applied to the surface of the electrodes for 1 minute followed by spin coating and rinsing by fresh 2-propanol, followed by drying on a hotplate at 100 °C. The organic semiconductor (OSC) formulation was spin coated onto the SD electrodes using a Laurell spinner set at 1500 rpm followed by baking on a hotplate for 60 seconds at 100°C. A solution of Hyflon® AD SF (9.2% w/w) commercialized by Solvay Specialty Polymers was spin coated at 3000 rpm and the samples were baked on a hotplate for 60s at 100°C. Gate electrodes were defined by evaporation of Aluminum (60nm) through a shadow mask in a thermal evaporator system.

**OTFT characterization**

OTFTs were tested using a Cascade Microtech EP6 DC probe station in conjunction with an Agilent B1500A semiconductor parameter analyzer. The Agilent system calculated the linear mobility according to the equation shown below (Equation 2)

$$\mu_{Lin} = \frac{\partial I_{DS}}{\partial V_G}\frac{L}{WC_iV_{DS}} \qquad \text{(Equation 2)}$$

where L is the transistor length, W is the transistor width and $C_i$ is the dielectric capacitance per unit area. $V_{DS}$ was set at -4V unless otherwise stated. The mobility values reported are an average of the 5 highest points in accumulation for each transistor. The standard deviation of the mobility values is reported as a percentage of the mean.

**Comparative Example 1 :** 1,4,8,11-tetramethyl-6,13-Triethylsilylethynylpentacene without binder; TG OTFT

The OTFT array fabricated and characterized as the Comparative Example 1 was fabricated and tested as described above. The formulation tested in the comparative example 1 included small molecule semiconductor however this formulation did not incorporate any binder.

1,4,8,11-tetramethyl-6,13-triethylsilylethynylpentacene represented by formula **(11)** was formulated in tetralin at 1.5% by weight total solids content. This was coated as an OSC layer in an OTFT device according to the method shown above for glass substrate.

The TFT performance of this formulation is shown below:

**μ Lin = 1.8±0.8 $cm^2$/Vs; standard deviation as a % of the mean value of the mobility was 44.4 %.**

The OTFT array fabricated and characterized as the Comparative Example 2 was fabricated and tested as described above. The formulation tested in the comparative example 2 included a small molecule semiconductor and a triarylamine polymer as binder.

**Comparative Example 2 :** 1,4,8,11-tetramethyl-6,13-Triethylsilylethynylpentacene with triarylamine polymer binder; TG OTFT

1,4,8,11-tetramethyl-6,13-triethylsilylethynylpentacene represented by formula **(11)** and Poly[bis(4-phenyl)(2,4,6-trimethylphenyl)amine] were formulated in tetralin in a ratio 50:50 at total solids content of 2% by weight. This was coated as an OSC layer in an OTFT device according to the method shown above for glass substrate.

The TFT performance of this formulation is shown below: **μ Lin = 3.8±1.1 $cm^2$/Vs; standard deviation as a % of the mean value of the mobility was 28.9 %.**

**Examples 1 to 5**

The OTFT arrays fabricated and characterized as the Examples 1 to 2 were fabricated and tested as described above. The formulations tested in the examples 1 to 6 included small molecule semiconductor and an amorphous small-molecule as binder. In these examples the ratio of binder to the semiconductor is in parts by weight.

**Example 1 :** 1,4,8,11-tetramethyl-6,13-Triethylsilylethynylpentacene with small-molecule bearing at least one optionally substituted carbazole moiety as the binder; TG OTFT

Binder 1, was formulated with 1,4,8,11-tetramethyl-6,13-triethylsilylethynyl pentacene represented by formula **(11)** in a ratio 60:40 at a total solids content of 1.5% by weight in tetralin and coated as an OSC layer in an OTFT device according to the method shown above for glass substrate devices.

The TFT performance of this formulation is shown below: **μ Lin = 6.6±0.3 $cm^2$/Vs; standard deviation as a % of the mean value of the mobility was 4.5 %.**

**Example 2 :** 1,4,8,11-tetramethyl-6,13-Triethylsilylethynylpentacene with small-molecule bearing at least one optionally substituted carbazole moiety as the binder; TG OTFT

Binder 1, was formulated with 1,4,8,11-tetramethyl-6,13-Triethylsilylethynylpentacene represented by formula **(11)** in a ratio 60:40 at a total solids content of 1.5% by weight in anisole and coated as an OSC layer in an OTFT device according to the method shown above for glass substrate devices.

The TFT performance of this formulation is shown below: **μ Lin = 15.1±0.4 $cm^2/Vs$; standard deviation as a % of the mean value of the mobility was 7.8 %.**

**Example 3 :** 1,4,8,11-tetramethyl-6,13-Triethylsilylethynylpentacene with small-molecule bearing at least one optionally substituted carbazole moiety as the binder; TG OTFT

Binder 2, was formulated with 1,4,8,11-tetramethyl-6,13-Triethylsilylethynylpentacene represented by formula **(11)** in a ratio 60:40 at a total solids content of 1.5% by weight in tetralin and coated as an OSC layer in an OTFT device according to the method shown above for glass substrate devices.

The TFT performance of this formulation is shown below: **μ Lin = 5.2±0.3 $cm^2/Vs$; standard deviation as a % of the mean value of the mobility was 5.8 %.**

**Example 4:** 1,4,8,11-tetramethyl-6,13-Triethylsilylethynylpentacene with small-molecule bearing at least one optionally substituted carbazole moiety as the binder; TG OTFT

Binder 2, was formulated with 1,4,8,11-tetramethyl-6,13-Triethylsilylethynylpentacene represented by formula **(11)** in a ratio 60:40 at a total solids content of 1.5% by weight in anisole and coated as an OSC layer in an OTFT device according to the method shown above for glass substrate devices.

The TFT performance of this formulation is shown below: **μ Lin = 5.2±0.3 $cm^2/Vs$; standard deviation as a % of the mean value of the mobility was 5.8 %.**

**Example 5 :** 1,4,8,11-tetramethyl-6,13-Triethylsilylethynylpentacene with small-molecule bearing at least one optionally substituted carbazole moiety as the binder; TG OTFT

Binder 3, was formulated with 1,4,8,11-tetramethyl-6,13-Triethylsilylethynylpentacene represented by formula **(11)** in a ratio 60:40 at a total solids content of 1.5% by weight in tetralin and coated as an OSC layer in an OTFT device according to the method shown above for glass substrate devices.

The TFT performance of this formulation is shown below: **μ Lin = 6.3±0.4 $cm^2/Vs$; standard deviation as a % of the mean value of the mobility was 6.3 %.**

The previous examples show that the organic semiconductor compositions in accordance with the present invention, comprising an organic semiconductor and a small molecule bearing at least one optionally substituted carbazole moiety as the binder yield OTFTs with improved performance compared to OTFTs obtained using compositions in accordance with the prior art. More particularly, measured linear mobility values were high, the standard deviation of the mobility values was low and the bias stress stability was improved.

## Claims

1. Composition comprising at least one crystalline organic semiconductor and at least one compound bearing at least one optionally substituted carbazole moiety, said compound corresponding to formula (a)

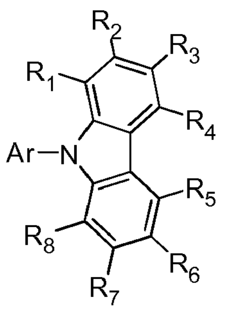

Formula (a)

and said compound being free of any spirobifluorene moiety corresponding to formula (b)

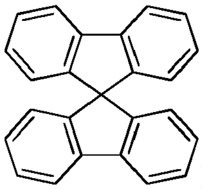

Formula (b)

wherein:

$R_1$, $R_2$, $R_3$, $R_4$, $R_5$, $R_6$, $R_7$ and $R_8$, which may be the same or different, represent H or a $C_1$-$C_{24}$ hydrocarbyl or heterohydrocarbyl group, and
Ar is an optionally substituted aryl or heteroaryl group.

**2.** Composition according to claim 1 wherein the compound bearing at least one optionally substituted carbazole moiety has a molecular weight of 1600 Daltons or less.

**3.** Composition according to claim 1 or 2 wherein the crystalline semiconductor has a field effect mobility of at least 0.01 $cm^2/Vs$.

**4.** Composition according to any one of the preceding claims wherein the weight ratio of crystalline organic semiconductor to compound bearing at least one optionally substituted carbazole moiety is comprised from 5/95 to 95/5.

**5.** Composition according to any one of the preceding claims which is essentially free of solvent.

**6.** Composition according to any one of the preceding claims but 5 additionally comprising at least one organic solvent.

**7.** Use of the composition according to anyone of claims 1 to 6 for the manufacture of a semiconducting layer.

**8.** Process for the manufacture of an organic semiconducting layer comprising applying the composition according to any one of claims 1 to 6 to form a layer onto a substrate.

**9.** Process according to claim 8, wherein the composition is the composition according to claim 6 and the process further comprises evaporating the solvent from the layer.

**10.** Device comprising the composition according to any one of claims 1 to 5 or the organic semiconducting layer manufactured by the process according to claim 8 or 9.

**11.** Device according to claim 10, which is or comprises a field effect transistor.

**12.** Device according to claim 11, which is a field effect transistor.

**13.** Display comprising the device according to claim 12.

Europäisches Patentamt
European Patent Office
Office européen des brevets

# EUROPEAN SEARCH REPORT

Application Number
EP 16 17 4440

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2016 025203 A (FUJIFILM CORP) 8 February 2016 (2016-02-08) * tables 1, 2 * * paragraph [0063] - paragraph [0074] * * paragraph [0115] * * tables 13, 14, 15 * * claims 1-25 * * the whole document * ----- | 1-13 | INV. C07D403/04 C07D405/04 C07D409/04 C07D495/04 C07D495/14 C07D209/86 C07D209/88 H01L51/05 H01L51/30 H01L51/40 |
| X | US 2013/207089 A1 (CHO HYUNDUCK [KR] ET AL) 15 August 2013 (2013-08-15) * paragraph [0028] * * the whole document * ----- | 1-13 | |
| X | WO 2008/069756 A1 (AGENCY SCIENCE TECH & RES [SG]; CHEN ZHIKUAN [SG]; ZHEN CHANGGUA [SG]) 12 June 2008 (2008-06-12) * paragraph [0012] * * paragraph [0077] - paragraph [0086] * * compounds 1-10 * * figure 2 * * claims 1, 18, 20, 21-23 * ----- | 1-13 | TECHNICAL FIELDS SEARCHED (IPC) |
| X | US 2012/091887 A1 (OSAKA HARUE [JP] ET AL) 19 April 2012 (2012-04-19) * paragraph [0222] - paragraph [0230] * * the whole document * ----- | 1-13 | C07D H01L |
| A | M-T LEE ET AL: "EFFICIENT GREEN COUMARIN DOPANTS FOR ORGANIC LIGHT-EMITTING DEVICES", ORGANIC LETTERS, AMERICAN CHEMICAL SOCIETY, US, vol. 6, no. 8, 15 April 2004 (2004-04-15), pages 1241-1244, XP002435446, ISSN: 1523-7060, DOI: 10.1021/OL049903D * C-545T; table 1 * ----- | 1-13 | |

The present search report has been drawn up for all claims

1

| Place of search | Date of completion of the search | Examiner |
|---|---|---|
| Munich | 21 September 2016 | Sarakinos, Georgios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 17 4440

This annex lists the patent family members relating to the patent documents cited in the above-mentioned European search report. The members are as contained in the European Patent Office EDP file on
The European Patent Office is in no way liable for these particulars which are merely given for the purpose of information.

21-09-2016

| Patent document cited in search report | Publication date | Patent family member(s) | Publication date |
|---|---|---|---|
| JP 2016025203 A | 08-02-2016 | NONE | |
| US 2013207089 A1 | 15-08-2013 | KR 101306192 B1 | 16-10-2013 |
| | | US 2013207089 A1 | 15-08-2013 |
| | | US 2016072090 A1 | 10-03-2016 |
| WO 2008069756 A1 | 12-06-2008 | CN 101573324 A | 04-11-2009 |
| | | EP 2121573 A1 | 25-11-2009 |
| | | JP 5121848 B2 | 16-01-2013 |
| | | JP 2010511700 A | 15-04-2010 |
| | | KR 20090097862 A | 16-09-2009 |
| | | TW 200837037 A | 16-09-2008 |
| | | US 2010133519 A1 | 03-06-2010 |
| | | WO 2008069756 A1 | 12-06-2008 |
| US 2012091887 A1 | 19-04-2012 | JP 5859269 B2 | 10-02-2016 |
| | | JP 2013060411 A | 04-04-2013 |
| | | JP 2016096350 A | 26-05-2016 |
| | | US 2012091887 A1 | 19-04-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 16014980 A
- US 2010200841 A
- WO 2005055248 A


### Non-patent literature cited in the description


- **JIANG et al.** *Advanced materials,* 2011, vol. 23 (43), 5075-5080
- **SVETLICHNYI et al.** *Semiconductors,* 2010, vol. 44 (12), 1581-1587
- **BOUDREAULT et al.** *J.Mater.Chem.,* 2009, vol. 19, 2921-2928
- **LI et al.** *Advanced Materials,* 2005, vol. 17 (7), 849-853
- **BOUDREAULT et al.** *Organic Electronics,* 2010, vol. 11 (10), 1649-1659
- **PARK et al.** *Advanced Materials,* 2013, vol. 25 (24), 3351-3356
- **LI et al.** *Organic Electronics,* 2009, vol. 10 (5), 910-917
- **DAOBEN ZHU et al.** *Chemical Reviews,* 2012, vol. 112, 2208-2267
- *Adv.Mater.,* 2013, vol. 25, 6392-6397
- *Chem. Comm.,* 2014, vol. 50, 5342-5344
- *Chem. Comm.,* 2013, vol. 49, 6483-6485